# EUROPEAN PATENT APPLICATION

(11) **EP 4 831 276 A1**
(43) Date of publication of application: **28.10.2026**
(21) Application number: 24907253.9
(22) Date of filing: 10.12.2024
(51) Int. Cl.: C12N 1/21, C12N 9/02, C12N 9/16, C12N 15/53, C12N 15/55, C12P 7/02

(54) **ALIPHATIC ALCOHOL-PRODUCING TRANSFORMANT OF BACTERIUM BELONGING TO GENUS HYDROGENOPHILUS**

(30) Priority: 21.12.2023 JP 2023215838
(71) Applicant: Utilization of Carbon Dioxide Institute Co.,Ltd., Tokyo 135-0091 (JP)
(72) Inventor: YUKAWA, Hideaki, Tokyo 135-0091 (JP)
(74) Representative: Kraus & Lederer PartGmbB
(86) International application number: PCT/JP2024/043666
(87) International publication number: WO 2025/134868

(57) **Abstract**

*Hydrogenophilus* bacteria lack a gene encoding an enzyme that catalyzes a reaction for producing an aliphatic alcohol, and hence the bacteria do not naturally produce an aliphatic alcohol. However, the bacteria start to produce an aliphatic alcohol when a gene encoding a thioesterase that produces a fatty acid from an acyl-ACP and a gene encoding an aliphatic acyl-CoA reductase that produces an aliphatic alcohol from an aliphatic acyl-CoA is introduced thereinto. Further, when the gene encoding the acyl-CoA dehydrogenase on the chromosome of the *Hydrogenophilus* bacteria is disrupted, production efficiency of the aliphatic alcohol is improved.

## Description

### Technical Field

The present invention relates to a *Hydrogenophilus* bacterium transformant having ability to produce aliphatic alcohol, and to a method for producing aliphatic alcohol using the same.

### Background Art

The Paris Agreement, which was adopted in 2015, provides that global emissions of greenhouse gases should be promptly reduced. Under the Agreement, Japan set the goal of reducing her emission of greenhouse gases such as carbon dioxide and methane by 46% compared with year 2013 levels, by the year 2030.

Globally, most chemical production depends on petroleum resources and thus contributes to increased greenhouse gas emissions. Accordingly, reducing dependence on petroleum is a desirable strategy for chemical production, and research and development of biorefineries, which produce green chemicals from biomass, are being actively conducted in various countries. However, biomass saccharification to produce raw materials for microbial fermentation requires complex processes and is also costly. In addition, the use of biomass that can serve as food or feed for chemical production disrupts the stable supply of food and feed. Furthermore, the large-scale use of biomass for chemical production may lead to environmental degradation.

As part of research on decarbonization, gases such as carbon dioxide, methane, and carbon monoxide have attracted attention as more sustainable carbon sources, and techniques for producing valuable substances and biofuels using microorganisms that utilize these gases are the subject of interest. In particular, carbon fixation and efficient utilization of carbon dioxide, a significant contributor to global warming, is highly anticipated.

### Background Art

Here, an aliphatic alcohol has been widely utilized as a raw material for detergents, an additive for cosmetics, pharmaceuticals, foods, and the like, a diesel fuel, and the like.

Raw materials for the aliphatic alcohol are mainly natural oils and fats in plants. Among plant oils and fats serving as raw materials, palm oil extracted from oil palm fruits is consumed the most in the world. In recent years, in Indonesia and Malaysia, which are major producing countries of palm oil, large-scale development of oil palm plantations has been performed to meet a rapidly increasing demand for oils and fats. However, the large-scale development of the oil palm plantations is accompanied by social problems, such as deforestation, loss of biodiversity, and forced labor. In 2004, the Roundtable on Sustainable Palm Oil (RSPO) was established to promote sustainable production and utilization of palm oil.

Meanwhile, in recent years, an attempt using a microorganism has been reported as an agricultural product-independent method of producing an aliphatic alcohol. Many microorganisms synthesize a fatty acyl-acyl carrier protein (acyl-ACP), which is the final product, through fatty acid synthesis, but do not have a gene group involved in aliphatic alcohol synthesis. For this reason, there have been proposed various methods of producing an aliphatic alcohol by culturing a transformant obtained by introducing an exogenous gene encoding an enzyme for forming this metabolic pathway into a host such as *Escherichia coli*.

For example, Non Patent Literature 1 reports an attempt to cause *Escherichia coli* to highly produce an aliphatic alcohol by: introducing a thioesterase gene derived from *Escherichia coli* in which a DNA region encoding a periplasmic transition signal sequence is deleted and an aliphatic acyl-CoA reductase gene derived from *Acinetobacter calcoaceticus* into *Escherichia coli*; and disrupting the fatty acid-degrading enzyme gene of *Escherichia coli* serving as a host.

Non Patent Literature 2 also reports, as an attempt similar to that of Non Patent Literature 1, an attempt to cause *Escherichia coli* to highly produce an aliphatic alcohol by: introducing a thioesterase gene derived from *Escherichia coli* in which a DNA region encoding a signal sequence is deleted and an aliphatic acyl-CoA reductase gene derived from *Marinobacter aquaeolei* into *Escherichia coli*; and disrupting the fatty acid-degrading enzyme gene of *Escherichia coli* serving as a host.

However, in each of the methods of Non Patent Literatures 1 and 2, biomass-derived glucose or starch is used as a carbon source. As described above, there are problems in that the use of biomass requires complex processes for converting biomass into sugars and thus is costly, and the industrial use of biomass has adverse environmental impacts despite its intended purpose.

In addition, Patent Literature 1 teaches a method of producing an aliphatic alcohol by using a transformant obtained by introducing the gene of an aliphatic alcohol synthase into *Synechocystis* sp. PCC6803 strain. The method is a method of producing an aliphatic alcohol by using cyanobacteria, which are photosynthetic bacteria, as a host.

The method including using cyanobacteria as a host has not been practically used as an industrial method of producing an aliphatic alcohol because the cyanobacteria have a higher carbon dioxide fixation ability than those of plants, but do not have a sufficient carbon dioxide fixation ability.

### Citation List

### Patent Literature

[Patent Literature 1] JP 2018-143192 A

### Non-Patent Literature

[Non Patent Literature 1] Stee EJ, et al. 2010. Microbial production of fatty-acid derived fuels and chemicals from plant biomass. Nature. 463: 559-562.
[Non Patent Literature 2] Liu A, et al.,2013. Fatty alcohol production in engineered E. coli expressing Marinobacter fatty acyl-CoA reductases. Appl microbiol Biotechnol. 97: 7061-7071.

### Summary of Invention

### Technical Problem

The objective of the present invention is to provide a transformant of a *Hydrogenophilus* bacterium that is capable of efficiently producing aliphatic alcohol utilizing carbon dioxide as a sole carbon source, and a method for efficiently producing aliphatic alcohol using this transformant.

### Solution to Problem

The present inventor has conducted extensive investigations to solve the above-mentioned problems and has obtained the following findings.
(i) *Hydrogenophilus* bacteria lack a gene encoding an enzyme that catalyzes a reaction for producing an aliphatic alcohol, and hence the bacteria do not naturally produce an aliphatic alcohol. However, the bacteria start to produce an aliphatic alcohol when a gene encoding an enzyme that catalyzes a reaction for producing an aliphatic alcohol is introduced thereinto. Specifically, when a gene encoding a thioesterase that produces a fatty acid from an acyl-ACP and a gene encoding an aliphatic acyl-CoA reductase that produces an aliphatic alcohol from an aliphatic acyl-CoA are introduced into the *Hydrogenophilus* bacteria, these genes are expressed in the *Hydrogenophilus* bacteria to impart an aliphatic alcohol production ability thereto.
(ii) A precursor of an aliphatic alcohol is decomposed, and hence the production efficiency of the aliphatic alcohol reduces because a gene encoding an acyl-CoA dehydrogenase is present on the chromosome of the *Hydrogenophilus* bacteria. When the gene encoding the acyl-CoA dehydrogenase of the *Hydrogenophilus* bacteria is disrupted, the decomposition of the precursor of the aliphatic alcohol is suppressed, and hence the production efficiency of the aliphatic alcohol is improved. Thus, the *Hydrogenophilus* bacteria start to produce the aliphatic alcohol extremely efficiently.

The present invention has been completed on the basis of the above-mentioned findings and provides the following [1] to [7].
[1] A transformant obtained by introducing, into a *Hydrogenophilus* bacterium, a gene encoding a thioesterase that produces a fatty acid from an acyl-ACP and a gene encoding an aliphatic acyl-CoA reductase that produces an aliphatic alcohol from an aliphatic acyl-CoA.
[2] The transformant according to [1], wherein the gene encoding the thioesterase that produces a fatty acid from an acyl-ACP is the following DNA (a1), (a2), (a3), (a4), or (a5).
   (a1) A DNA comprising the nucleotide sequence of SEQ ID NO: 1
   (a2) A DNA comprising a nucleotide sequence that has 90% or more identity to SEQ ID NO: 1 and encoding a polypeptide having thioesterase activity for producing a fatty acid from an acyl-ACP (a3) A DNA encoding a polypeptide comprising the amino acid sequence of SEQ ID NO: 2
   (a4) A DNA encoding a polypeptide comprising an amino acid sequence that has 90% or more identity to SEQ ID NO: 2, the polypeptide having thioesterase activity for producing a fatty acid from an acyl-ACP
   (a5) A DNA encoding a polypeptide comprising an amino acid sequence that has 1 to 20 amino acids deleted, substituted, inserted, or added in the amino acid sequence of SEQ ID NO: 2, the polypeptide having thioesterase activity for producing a fatty acid from an acyl-ACP
[3] The transformant according to [1] or [2], wherein the gene encoding the aliphatic acyl-CoA reductase that produces an aliphatic alcohol from an aliphatic acyl-CoA is the following DNA (b1), (b2), (b3), (b4), or (b5).
   (b1) A DNA comprising the nucleotide sequence of SEQ ID NO: 3
   (b2) A DNA comprising a nucleotide sequence that has 90% or more identity to SEQ ID NO: 3 and encoding a polypeptide having aliphatic acyl-CoA reductase activity for producing an aliphatic alcohol from an aliphatic acyl-CoA
   (b3) A DNA encoding a polypeptide comprising the amino acid sequence of SEQ ID NO: 4
   (b4) A DNA encoding a polypeptide comprising an amino acid sequence that has 90% or more identity to SEQ ID NO: 4, the polypeptide having aliphatic acyl-CoA reductase activity for producing an aliphatic alcohol from an aliphatic acyl-CoA
   (b5) A DNA encoding a polypeptide comprising an amino acid sequence that has 1 to 50 amino acids deleted, substituted, inserted, or added in the amino acid sequence of SEQ ID NO: 4, the polypeptide having aliphatic acyl-CoA reductase activity for producing an aliphatic alcohol from an aliphatic acyl-CoA
[4] The transformant according to any one of [1] to [3], wherein an acyl-CoA dehydrogenase gene on a chromosome of the *Hydrogenophilus* bacterium is disrupted.
[5] The transformant according to [4], wherein the acyl-CoA dehydrogenase gene is the following DNA (c1), (c2), (c3), (c4), or (c5).
   (c1) A DNA comprising the nucleotide sequence of SEQ ID NO: 5
   (c2) A DNA comprising a nucleotide sequence that has 90% or more identity to SEQ ID NO: 5 and encoding a polypeptide having acyl-CoA dehydrogenase activity
   (c3) A DNA encoding a polypeptide comprising the amino acid sequence of SEQ ID NO: 6
   (c4) A DNA encoding a polypeptide comprising an amino acid sequence that has 90% or more identity to SEQ ID NO: 6, the polypeptide having acyl-CoA dehydrogenase activity
   (c5) A DNA encoding a polypeptide comprising an amino acid sequence that has 1 to 80 amino acids deleted, substituted, inserted, or added in the amino acid sequence of SEQ ID NO: 6, the polypeptide having acyl-CoA dehydrogenase activity
[6] The transformant according to any one of [1] to [5], wherein the *Hydrogenophilus* bacterium is *Hydrogenophilus thermoluteolus*.
[7] A method of producing an aliphatic alcohol, comprising a step of culturing the transformant according to any one of [1] to [6].

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical products can be produced while suppressing global warming attributed to increased carbon dioxide emissions.

Bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, such bacteria grow slowly, but the growth rate of hydrogen-oxidizing bacteria, *Hydrogenophilus* bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

The *Hydrogenophilus* bacteria have a series of genes encoding enzymes that catalyze the production of an acyl-ACP, which is the final product of fatty acid synthesis, like many microorganisms, but do not have a series of genes encoding enzymes that catalyze a reaction for producing an aliphatic alcohol from an acyl-ACP.

The present inventor has found that it is difficult to express a protein that functions even when an exogenous gene is introduced into the *Hydrogenophilus* bacteria. However, according to the present invention, when a gene encoding a thioesterase that produces a fatty acid from an acyl-ACP and a gene encoding an aliphatic acyl-CoA reductase that produces an aliphatic alcohol from an aliphatic acyl-CoA are introduced into the *Hydrogenophilus* bacteria, these genes are expressed in the *Hydrogenophilus* bacteria, and hence the *Hydrogenophilus* bacteria become capable of producing an aliphatic alcohol by using carbon dioxide as a sole carbon source.

Wild-type *Hydrogenophilus* bacteria each have an acyl-CoA dehydrogenase gene, which is a fatty acid-degrading enzyme gene, on its genome, and have a fatty acid degradation ability by producing an active acyl-CoA dehydrogenase. When a gene encoding an acyl-CoA dehydrogenase, which is a fatty acid-degrading enzyme, is disrupted, the decomposition of a fatty acid, which is a precursor of an aliphatic alcohol, is suppressed, and hence the productivity of the aliphatic alcohol is improved.

As described above, the *Hydrogenophilus* bacteria each have an atypically remarkable carbon dioxide fixation ability among organisms having a carbon dioxide fixation ability. Accordingly, the use of the transformant of the present invention enables industrial-scale production of an aliphatic alcohol through fixation of carbon dioxide.

An aliphatic alcohol has been widely used as a raw material for detergents, an additive for cosmetics, pharmaceuticals, foods, and the like, a diesel fuel, and the like. Various problems caused by producing a large amount of palm oil, which is a raw material for an aliphatic alcohol, can be solved because the aliphatic alcohol can be produced on an industrial scale by using the transformant of the present invention.

### Brief Description of Drawings

[Figure 1] Figure 1 is a gel photograph of SDS-PAGE showing that a disrupted cell solution of a strain obtained by transforming *Hydrogenophilus thermoluteolus* with a thioesterase gene degraded C12:0-acyl-ACP.
[Figure 2] Figure 2 is a chromatogram of gas chromatography showing that a disrupted cell solution of a strain obtained by transforming *Hydrogenophilus thermoluteolus* with an aliphatic acyl-CoA reductase gene produced 1-dodecanol from lauroyl-CoA and produced 1-tetradecanol from myristoyl-CoA.

### Description of Embodiments

The present invention is described in detail below.

### (1) Transformant having aliphatic alcohol production ability

A transformant (transformed cell) of the present invention is a transformant obtained by introducing, into a *Hydrogenophilus* bacterium serving as a host, a gene encoding a thioesterase that produces a fatty acid from an acyl-ACP and a gene encoding an aliphatic acyl-CoA reductase that produces an aliphatic alcohol from an aliphatic acyl-CoA. In other words, the transformant of the present invention is a *Hydrogenophilus* bacterium having an exogenous gene encoding thioesterase that produces a fatty acid from an acyl-ACP and an exogenous gene encoding aliphatic acyl-CoA reductase that produces an aliphatic alcohol from an aliphatic acyl-CoA.

In the thioesterase in the present invention that produces a fatty acid from an acyl-ACP, the range of an acyl group of the acyl-ACP that can serve as a substrate may vary depending on the amino acid sequence of the thioesterase. However, for example, an acyl-ACP having a linear or branched, saturated or unsaturated acyl group having 8 to 18 carbon atoms (in particular, any number of carbon atoms of from 8 to 18) can serve as a substrate. In addition, in the aliphatic acyl-CoA reductase in the present invention that produces an aliphatic alcohol from an aliphatic acyl-CoA, the range of an aliphatic acyl group of the aliphatic acyl-CoA that can serve as a substrate may vary depending on the amino acid sequence of the aliphatic acyl-CoA reductase. However, for example, an aliphatic acyl-CoA having a linear or branched, saturated or unsaturated aliphatic acyl group having 12 to 18 carbon atoms (in particular, any number of carbon atoms of from 8 to 18) can serve as a substrate.

The above-mentioned thioesterase gene and aliphatic acyl-CoA reductase gene may be the DNA of a thioesterase gene and the DNA of an aliphatic acyl-CoA reductase gene isolated from naturally occurring bacteria, respectively, or may each be a DNA artificially synthesized by using an approach known to those skilled in the art.

### Thioesterase gene

The gene of the thioesterase that produces a fatty acid from an acyl-ACP does not need to have been previously identified as a gene of such thioesterase, and may be any DNA encoding a polypeptide having such thioesterase activity.

In the present invention, the fact that a polypeptide has thioesterase activity using an acyl-ACP as a substrate is recognized by: reacting a test polypeptide with an RCO-ACP (R represents a linear saturated alkyl group having 11 carbon atoms); and recognizing by SDS-PAGE that the RCO-ACP is decomposed and consumed. Specifically, a disrupted cell solution prepared by a method described in the section "Examples" is used as a crude enzyme solution and reacted with the RCO-ACP of *Escherichia coli* (R represents a linear saturated alkyl group having 11 carbon atoms), and the disappearance of a band of the RCO-ACP is recognized by subjecting a reaction product to SDS-PAGE. In the method, the consumption of the RCO-ACP indicates that the test polypeptide has such activity as to decompose an acyl-ACP to produce a fatty acid.

Preferred examples of the above-mentioned thioesterase gene include the thioesterase gene of *Tepidiphilus thermophilus*, in particular, the thioesterase gene of *Tepidiphilus thermophilus* in which a DNA region encoding an N-terminal peptide presumed to be a periplasmic transition signal sequence is deleted. The thioesterase gene of *Tepidiphilus thermophilus* in which the putative signal sequence is deleted is a DNA consisting of the nucleotide sequence of SEQ ID NO: 1.

In addition, there may be used a DNA comprising a nucleotide sequence that has 90% or more, even 95% or more, even 98% or more, even 99% or more identity to SEQ ID NO: 1 (in particular, consisting of a nucleotide sequence that has 90% or more, even 95% or more, even 98% or more, even 99% or more identity to SEQ ID NO: 1) and encoding a polypeptide having thioesterase activity for producing a fatty acid from an acyl-ACP.

Preferred examples of the above-mentioned thioesterase gene also include a DNA encoding the thioesterase of *Tepidiphilus thermophilus*, in particular, a DNA encoding the thioesterase of *Tepidiphilus thermophilus* in which an N-terminal peptide presumed to be a periplasmic transition signal sequence is deleted. The thioesterase of *Tepidiphilus thermophilus* in which the putative signal sequence is deleted is a polypeptide consisting of the amino acid sequence of SEQ ID NO: 2.

In addition, there may be used a DNA encoding a polypeptide comprising an amino acid sequence that has 90% or more, even 95% or more, even 98% or more, even 99% or more identity to SEQ ID NO: 2 (in particular, consisting of an amino acid sequence that has 90% or more, even 95% or more, even 98% or more, even 99% or more identity to SEQ ID NO: 2), the polypeptide having thioesterase activity for producing a fatty acid from an acyl-ACP.

Further, there may be used a DNA encoding a polypeptide comprising an amino acid sequence that has 1 to 20, 1 to 10, 1 to 5, or 1 to 3 amino acids, or 1 amino acid deleted, substituted, inserted, or added in the amino acid sequence of SEQ ID NO: 2 (in particular, consisting of an amino acid sequence that has 1 to 20, 1 to 10, 1 to 5, or 1 to 3 amino acids, or 1 amino acid deleted, substituted, inserted, or added in the amino acid sequence of SEQ ID NO: 2), the polypeptide having thioesterase activity for producing a fatty acid from an acyl-ACP.

### Aliphatic acyl-CoA reductase gene

The aliphatic acyl-CoA reductase gene does not need to have been previously identified as an aliphatic acyl-CoA reductase gene, and may be any DNA encoding a polypeptide having aliphatic acyl-CoA reductase activity.

The fact that a test polypeptide has aliphatic acyl-CoA reductase activity using an aliphatic acyl-CoA as a substrate is recognized by: reacting the test polypeptide with lauroyl-CoA in the presence of NADPH; and detecting produced 1-dodecanol with a gas chromatograph or the like.

Preferred examples of the aliphatic acyl-CoA reductase gene include the aliphatic acyl-CoA reductase gene of *Marinobacter lutaoensis*. The aliphatic acyl-CoA reductase gene of *Marinobacter lutaoensis* is a DNA consisting of the nucleotide sequence of SEQ ID NO: 3.

In addition, there may be used a DNA comprising a nucleotide sequence that has 90% or more, even 95% or more, even 98% or more, even 99% or more identity to SEQ ID NO: 3 (in particular, consisting of a nucleotide sequence that has 90% or more, even 95% or more, even 98% or more, even 99% or more identity to SEQ ID NO: 3) and encoding a polypeptide having aliphatic acyl-CoA reductase activity for producing an aliphatic alcohol from an aliphatic acyl-CoA.

Preferred examples of the aliphatic acyl-CoA reductase gene also include a DNA encoding the aliphatic acyl-CoA reductase of *Marinobacter lutaoensis*. The aliphatic acyl-CoA reductase of *Marinobacter lutaoensis* is a polypeptide consisting of the amino acid sequence of SEQ ID NO: 4.

In addition, there may be used a DNA encoding a polypeptide comprising an amino acid sequence that has 90% or more, even 95% or more, even 98% or more, even 99% or more identity to SEQ ID NO: 4 (in particular, consisting of an amino acid sequence that has 90% or more, even 95% or more, even 98% or more, even 99% or more identity to SEQ ID NO: 4), the polypeptide having aliphatic acyl-CoA reductase activity for producing an aliphatic alcohol from an aliphatic acyl-CoA.

Further, there may be used a DNA encoding a polypeptide comprising an amino acid sequence that has 1 to 50, 1 to 30, 1 to 10, 1 to 5, or 1 to 3 amino acids, or 1 amino acid deleted, substituted, inserted, or added in the amino acid sequence of SEQ ID NO: 4 (in particular, consisting of an amino acid sequence that has 1 to 50, 1 to 30, 1 to 10, 1 to 5, or 1 to 3 amino acids, or 1 amino acid deleted, substituted, inserted, or added in the amino acid sequence of SEQ ID NO: 4), the polypeptide having aliphatic acyl-CoA reductase activity for producing an aliphatic alcohol from an aliphatic acyl-CoA.

In view of codon degeneracy or codon preference in *Hydrogenophilus* bacteria, the DNA sequence encoding a protein comprising the amino acid sequence of SEQ ID NO: 2 or 4 may be subjected to a variety of nucleotide substitutions in the coding region, as long as such substitutions do not alter the amino acid sequence of the protein expressed from the coding region.

In the present invention, the identities of nucleotide sequences and amino acid sequences were calculated using GENETYX ver. 17 (GENETYX).

### Hydrogenophilus bacteria

Examples of *Hydrogenophilus* bacteria include *Hydrogenophilus thermoluteolus*, *Hydrogenophilus halorhabdus*, *Hydrogenophilus denitrificans*, *Hydrogenophilus hirschii*, *Hydrogenophilus islandicus*, *Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977))(It proliferates double per hour.). H*ydrogenophilus thermoluteolus* strain NBRC 14978 is internationally deposited under the Budapest Treaty, and is thus publicly available.

The hosts *Hydrogenophilus* bacteria may be bacteria isolated from nature or genetically modified bacteria derived from bacteria isolated from nature. Genetic modification may be intended, for example, to allow high expression of an introduced thioesterase gene and aliphatic acyl-CoA reductase gene.

Such a genetic modification can be made, for example, by removing (curing) endogenous plasmids in *Hydrogenophilus* bacteria, or other techniques. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Method for producing transformant

Next, methods for obtaining transformants by introducing thioesterase gene and aliphatic acyl-CoA reductase gene into *Hydrogenophilus* bacteria are described.

The introduction of these genes into *Hydrogenophilus* bacteria can be carried out using common methods for introducing exogenous genes into bacteria. These genes may be directly introduced into *Hydrogenophilus* bacteria. Alternatively, thioesterase gene and aliphatic acyl-CoA reductase gene may be incorporated into a vector for transformation such as a plasmid vector, viral vector, cosmid, fosmid, or BAC, and then, an obtained vector may be introduced into *Hydrogenophilus* bacteria.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 (SEQ ID NO: 7) is preferable. Those skilled in the art can prepare pCAMO-6 by utilizing a gene synthesis service or the like based on its DNA sequence.

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of thioesterase gene and aliphatic acyl-CoA reductase gene into a *Hydrogenophilus* bacterium (transformation) can be carried out by publicly known methods such as calcium chloride method, and electric pulse method (electroporation method).

### Disruption of acyl-CoA dehydrogenase gene

It is preferred that the transformant of the present invention include an exogenous thioesterase gene and an exogenous aliphatic acyl-CoA reductase gene, and an acyl-CoA dehydrogenase gene on the chromosome of its *Hydrogenophilus* bacterium be disrupted.

The thioesterase gene and the aliphatic acyl-CoA reductase gene may be introduced after the acyl-CoA dehydrogenase gene on the genome of the *Hydrogenophilus* bacterium has been disrupted, or the acyl-CoA dehydrogenase gene on the genome may be disrupted after the thioesterase gene and the aliphatic acyl-CoA reductase gene have been introduced into the *Hydrogenophilus* bacterium.

In the present invention, the term "disruption of an acyl-CoA dehydrogenase gene" means that the activity of an acyl-CoA dehydrogenase, which is a product of the gene, is lower than that of a parent strain in which the acyl-CoA dehydrogenase gene is not disrupted. It is preferred that the activity of the product of the gene become 50% or less, in particular 10% or less, in particular 1% or less of that of the parent strain.

As the expression level of a gene reduces, the activity of a gene product reduces. The expression level of the gene may be measured by a quantitative PCR method (qPCR) or a next-generation sequencing method (NGS). In addition, even when part of the coding region or expression-regulating region of the gene is mutated, the gene may be normally expressed. However, even such case corresponds to the "disruption of the gene" as long as the activity of the gene product is lower than that of its wild strain.

The disruption of a gene can be performed by mutating (the deletion, substitution, insertion, addition of nucleotide(s), or a combination thereof) the coding region of the gene or a gene expression-regulating region thereof such as a promoter region, the coding region or the gene expression-regulating region being present on the chromosome of a *Hydrogenophilus* bacterium. A method of disrupting the gene has been well known, and examples thereof include: a gene knockout method including using homologous recombination; and a screening method based on random introduction of gene mutation through use of a mutagen and on a phenotype.

For example, the following may be performed: a disrupted acyl-CoA dehydrogenase gene is produced by a PCR or the like; the disrupted gene is introduced into a parental strain; and homologous recombination between the disrupted gene and a gene on the genome of the parent strain is caused to substitute an acyl-CoA dehydrogenase gene on the chromosome of the parent strain with the disrupted gene.

A gene disruption method based on homologous recombination has been well known. The present inventor has found an rpsL gene as a gene responsible for streptomycin sensitivity that functions in *Hydrogenophilus* bacteria (in particular, in a streptomycin-resistant strain) and hence may be used as a counterselection marker. The use of the gene can disrupt the acyl-CoA dehydrogenase gene of *Hydrogenophilus* bacteria through homologous recombination.

A mutant of the present invention is preferably such that the coding region of an acyl-CoA dehydrogenase gene is mutated. To mutate the coding region, typically, the entire region of each gene or a partial region thereof may be deleted. In addition, the gene may be disrupted by inserting some nucleotide, oligonucleotide, or polynucleotide into the inside of the gene. In addition, the entire region or partial region of the gene may be substituted with another nucleotide, oligonucleotide, or polynucleotide. When the deletion, substitution, and/or insertion of a plurality of nucleotides is introduced, the mutation may be introduced at one site in the gene, or may be dispersedly introduced at a plurality of sites therein.

The number of nucleotides to be deleted, substituted, or inserted is preferably 3 or more, in particular 5 or more, in particular 10 or more, in particular 20 or more, in particular 50 or more. In addition, nucleotides whose number corresponds to 1% or more, in particular 5% or more, in particular 10% or more, in particular 50% or more of the full length of a gene are preferably deleted, substituted, or inserted. Thus, each gene can be reliably disrupted. The deletion or the substitution may be performed over the full length of the gene (i.e., 100% of nucleotides for forming the gene may be deleted or substituted). The number of nucleotides to be inserted may be set to 100,000 or less, 1,000 or less, or 100 or less.

A method of disrupting the acyl-CoA dehydrogenase gene is described in detail by taking, as an example, a method of disrupting the acyl-CoA dehydrogenase gene by the deletion of its entire coding region. First, a plasmid may be produced by: amplifying a DNA fragment containing a DNA of a region encoding the acyl-CoA dehydrogenase gene of a *Hydrogenophilus* bacterium, a DNA of the 5' upstream region of the region encoding the acyl-CoA dehydrogenase gene, and a DNA of the 3' downstream region thereof by a PCR method through use of the genomic DNA of the *Hydrogenophilus* bacterium as a template; and ligating the amplified DNA fragment with a marker cassette containing a streptomycin sensitivity gene (counterselection marker) that functions in the *Hydrogenophilus* bacterium and a kanamycin resistance gene (positive selection marker) that functions in the *Hydrogenophilus* bacterium.

To improve homologous recombination efficiency, it is preferred that the DNA of the 5' upstream region and the DNA of the 3' downstream region be each a DNA of 10 or more nucleotides, preferably 50 or more nucleotides, more preferably 100 or more nucleotides. Alternatively, the DNAs to be linked to each other do not need to consist of nucleotide sequences completely identical to the 5' upstream region of the region to be deleted and the 3' downstream region of the region to be deleted, respectively, and as the lengths of the regions to be used for homologous recombination increase, homologous recombination can be caused with lower identity to the 5' upstream region or 3' downstream region of the region to be deleted.

Next, a gene for disruption in which the acyl-CoA dehydrogenase gene is deleted is produced. A DNA fragment in which the acyl-CoA dehydrogenase gene is deleted may be amplified by a PCR through use of the above-mentioned plasmid DNA having the acyl-CoA dehydrogenase gene as a template, and cyclized by using T4 polynucleotide kinase and T4 DNA ligase.

Next, homologous recombination using the gene for disruption is performed. An operation of introducing the gene for disruption into the streptomycin-resistant strain of the *Hydrogenophilus* bacterium may be performed, and a strain that is streptomycin-resistant and has lost kanamycin resistance may be selected. Thus, a genetically modified organism in which the marker cassette inserted into the acyl-CoA dehydrogenase gene has been excised, that is, a genetically modified organism in which the acyl-CoA dehydrogenase gene is deleted is obtained.

A wild-type acyl-CoA dehydrogenase gene is, for example, any one of the following DNAs (c1) to (c5).
(c1) A DNA comprising the nucleotide sequence of SEQ ID NO: 5
(c2) A DNA comprising a nucleotide sequence that has 90% or more, even 95% or more, even 98% or more, even 99% or more identity to SEQ ID NO: 5 (in particular, consisting of a nucleotide sequence that has 90% or more, even 95% or more, even 98% or more, even 99% or more identity to SEQ ID NO: 5), and encoding a polypeptide having acyl-CoA dehydrogenase activity
(c3) A DNA encoding a polypeptide comprising the amino acid sequence of SEQ ID NO: 6
(c4) A DNA encoding a polypeptide comprising an amino acid sequence that has 90% or more, even 95% or more, even 98% or more, even 99% or more identity to SEQ ID NO: 6 (in particular, consisting of an amino acid sequence that has 90% or more, even 95% or more, even 98% or more, even 99% or more identity to SEQ ID NO: 6), the polypeptide having acyl-CoA dehydrogenase activity
(c5) A DNA encoding a polypeptide comprising an amino acid sequence that has 1 to 80, 1 to 50, 1 to 30, 1 to 10, 1 to 5, or 1 to 3 amino acids, or 1 amino acid deleted, substituted, inserted, or added in the amino acid sequence of SEQ ID NO: 6 (in particular, consisting of the amino acid sequence that has 1 to 80, 1 to 50, 1 to 30, 1 to 10, 1 to 5, or 1 to 3 amino acids, or 1 amino acid deleted, substituted, inserted, or added in the amino acid sequence of SEQ ID NO: 6), the polypeptide having acyl-CoA dehydrogenase activity

SEQ ID NO: 5 is the nucleotide sequence of an HPTL_0940 gene, which is the acyl-CoA dehydrogenase gene of the wild strain of *Hydrogenophilus thermoluteolus*, and SEQ ID NO: 6 is the amino acid sequence of a polypeptide encoded by the HPTL_0940 gene of the wild strain of *Hydrogenophilus thermoluteolus*.

The fact that a test polypeptide has acyl-CoA dehydrogenase activity is recognized by spectrophotometrically analyzing an oxidation-reduction reaction using ferrocenium hexafluorophosphate. The spectrophotometric analysis is performed by observing a change in absorption spectrum at 300 nm when octanoyl-CoA serving as a substrate is added to a mixed solution of a disrupted cell solution of *Escherichia coli* strain JM109 in which the test polypeptide is overexpressed and ferrocenium hexafluorophosphate. When the test polypeptide has acyl-CoA dehydrogenase activity, the reduction reaction of ferrocenium hexafluorophosphate occurs, and hence the reduction in absorption spectrum at 300 nm is detected. Specific reaction conditions may be appropriately set by those skilled in the art with reference to "Lodewijk I, et al., 1993. A simple spectrophotometric assay for long-chain acyl-CoA dehydrogenase activity measurements in human skin fibroblasts. Ann Clin Biochem. 30: 293-297."

### (2) Method for producing aliphatic alcohol

The present invention provides a method for producing aliphatic alcohol with use of the transformant of the present invention described above. The method includes a step of culturing the transformant of the present invention, especially, a step of culturing the transformant of the present invention using an inorganic or organic culture medium while supplying a gas containing carbon dioxide, preferably a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that aliphatic alcohol can be produced efficiently.

*Hydrogenophilus* bacteria can grow using hydrogen as a source of energy and using carbon dioxide as a sole carbon source, and thus, *Hydrogenophilus* bacteria can fix carbon dioxide efficiently when aliphatic alcohol is produced using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and aliphatic alcohol can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into an airtight culture container and shaking culture can be carried out, or the transformant can be cultured using an airtight culture container while introducing mixed gases into the culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and aliphatic alcohol can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and aliphatic alcohol can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and aliphatic alcohol can be produced efficiently.

An aliphatic alcohol is produced in the culture liquid by culturing the transformant as described above. The recovery of the culture liquid enables the recovery of the aliphatic alcohol. Further, the aliphatic alcohol may be separated from the reaction mixture by a known method. Examples of such known method include liquid separation and distillation.

### Examples

Next, the present invention is described with reference to Examples. The technical scope of the present invention is not limited by the following Examples.

### (1) Construction of gene expression plasmids

Gene expression plasmids were constructed by Gibson Assembly.

A DNA fragment of SEQ ID NO: 1, which was a thioesterase gene in which a DNA region encoding an N-terminal peptide presumed to be a periplasmic transition signal sequence was deleted (hereinafter sometimes referred to as "mutant thioesterase gene"), was amplified by a PCR through use of the genomic DNA of *Tepidiphilus thermophilus* as a template. The periplasmic transition signal sequence was estimated with SignalP 6.0 (https://services.healthtech.dtu.dk/services/SignalP-6.0/).

In addition, a DNA fragment of SEQ ID NO: 3, which was an aliphatic acyl-CoA reductase gene, was amplified by a PCR through use of the genomic DNA of *Marinobacter lutaoensis* as a template.

The PCRs were each performed by a conventional method through use of "2720 Thermal Cycler" manufactured by Thermo Fisher Scientific Inc. and through use of KOD FX Neo (manufactured by Toyobo Co., Ltd.) as a reaction reagent. After that, the resulting reaction mixture was subjected to electrophoresis on a 1% agarose gel, and the DNA fragment was recovered from the agarose gel by using GEL/PCR Purification Mini Kit (FAVORGEN).

The following primers were used for the PCRs.
Primers for amplifying mutant thioesterase gene of *Tepidiphilus thermophilus* (SEQ ID NO: 1)

| | | |
|---|---|---|
| (a-1) | 5'-GATCTGGAGGAGAAACGCATATGGCCACGATTCTCGTCTTCGGC-3' | (SEQ ID NO: 8) |
| (b-1) | 5'-ACGCGGCGCCGCCTCCGGGCGCTGAGAATTCGAGCTCCGTCGAC-3' | (SEQ ID NO: 9) |

A sequence homologous to that of vector pCAMO-6 is added to each of the primers (a-1) and (b-1).
Primers for amplifying aliphatic acyl-CoA reductase gene of *Marinobacter lutaoensis* (SEQ ID NO: 3)

| | | |
|---|---|---|
| (a-2) | 5'-GATCTGGAGGAGAAACGCATATGGCAACACCGCATGTATCCACC-3' | (SEQ ID NO: 10) |
| (b-2) | 5'-AGCGAGAAAGAAACAGGCCGCCTGAGAATTCGAGCTCCGTCGAC-3' | (SEQ ID NO: 11) |

A sequence homologous to that of vector pCAMO-6 is added to each of the primers (a-2) and (b-2).

The resulting reaction mixture was subjected to gel electrophoresis. As a result, an about 0.6-kbp DNA fragment for the mutant thioesterase gene and an about 1.5-kbp DNA fragment for the aliphatic acyl-CoA reductase gene were detected, and were recovered from the gel.

Plasmid vector pCAMO-6 was amplified by PCR through use of a DNA fragment of SEQ ID NO: 7 as a template for subsequent DNA ligation by Gibson Assembly. The following primers were used for the PCR.
Primers for amplifying plasmid vector pCAMO-6

| | | |
|---|---|---|
| (a-3) | 5'-GAATTCGAGCTCCGTCGACA-3' | (SEQ ID NO: 12) |
| (b-3) | 5'-ATGCGTTTCTCCTCCAGATC-3' | (SEQ ID NO: 13) |

The resulting reaction mixture was subjected to gel electrophoresis. As a result, an about 5.2-kbp DNA fragment corresponding to the vector gene was detected, and was recovered from the gel.

The above-mentioned DNA fragment of vector pCAMO-6 and the above-mentioned DNA fragment of the thioesterase gene or the above-mentioned DNA fragment of the aliphatic acyl-CoA reductase gene were ligated to each other with Gibson Assembly Master Mix manufactured by New England Biolabs.

*Escherichia coli* strain JM109 was transformed separately with each resulting reaction mixture by heat shock method, applied to LB medium containing 50 µg/mL kanamycin, and cultured at 37°C for 24 hours.

Each grown strain on the LB medium was inoculated with a platinum loop into 5 mL of liquid LB medium containing 50 µg/mL kanamycin in a test tube, and cultured with shaking at 37°C. Plasmid DNA was extracted from the culture liquid. The analysis of the nucleotide sequence of the gene inserted in each plasmid was outsourced to Eurofins Genomics K.K. and performed by Sanger method. The results revealed that the sequences were identical to the corresponding sequences in a database.

### (2) Production of introduced strain of mutant thioesterase gene or aliphatic acyl-CoA reductase gene

### (2-1) Gene introduction

*Hydrogenophilus thermoluteolus* strain TH-1 (NBRC 14978) (hereinafter sometimes referred to as "strain TH-1") was transformed with each plasmid obtained in the section "(1) Construction of gene expression plasmids" by electric pulse method (electroporation method), was seeded on solid LB medium containing 50 µg/mL kanamycin, and was cultured at 52°C for 24 hours. Each grown strain on the solid LB medium was inoculated with a platinum loop into solid LB medium containing 50 µg/mL kanamycin, and was cultured at 52°C for 24 hours. For each grown strain on the solid LB medium, the amplification of an inserted fragment of each plasmid was detected by PCR.

As a result, the amplification of a DNA fragment having a length corresponding to each gene was recognized. A strain TH-1 transformant obtained by transforming a plasmid containing the mutant thioesterase gene of *Tepidiphilus thermophilus* was named strain TE, and a strain TH-1 transformant obtained by transforming a plasmid containing the aliphatic acyl-CoA reductase gene of *Marinobacter lutaoensis* was named strain FAR.

### (2-2) Measurement of thioesterase activity

The mutant thioesterase gene-introduced strain of *Hydrogenophilus thermoluteolus* (strain TE) prepared as described above was inoculated with a platinum loop into liquid A medium containing 50 µg/mL kanamycin, and was cultured with shaking at 52°C for 24 hours while a gas mixture of H₂:O₂:CO₂ = 7.5:1:1.5 was supplied along with the culture. Bacterial cells were recovered from 2 mL of the culture liquid thus cultured by centrifugation (4°C, 5,000 g, 10 minutes). The bacterial cells were suspended in 1 mL of a reaction buffer (200 mM Tris-HCl (pH 7.5)), and a disrupted cell solution obtained by disrupting the bacterial cells with an ultrasonic wave was obtained. The solution was used as a crude enzyme solution.

For a thioesterase activity assay, C12:0-acyl-ACP of *Escherichia coli* containing a structure similar to that of C12:0-acyl-ACP of *Hydrogenophilus thermoluteolus* (C12:0-acyl represents a linear saturated acyl group having 12 carbon atoms) was prepared as a substrate. A crude purification liquid of C12:0-acyl-ACP was prepared by binding lauroyl-CoA to the ACP of *Escherichia coli* through use of the holo-ACP synthase of *Escherichia coli* as a catalyst. Specific preparation conditions may be appropriately set by those skilled in the art with reference to "Leonardo L, et al., 2016. A high yield optimized method for the production of acylated ACPs enabling the analysis of enzymes involved in P. falciparum fatty acid biosynthesis. Biochem Biophysics Reports. 8: 310-317.".

The crude enzyme solution and the crude purification liquid of C12:0-acyl-ACP having the same volume as that of the crude enzyme solution were mixed, and the mixture was subjected to a reaction at 52°C for 15 minutes. The reaction mixture was analyzed by SDS-PAGE, and was evaluated for its thioesterase activity by recognizing the disappearance of a band of C12:0-acyl-ACP due to hydrolysis.

For a negative control (NC) strain obtained by introducing empty vector pCAMO-6 into *Hydrogenophilus thermoluteolus* strain TH-1, the disrupted cell solution of cultured bacterial cells was similarly obtained, and was similarly evaluated for its thioesterase activity.

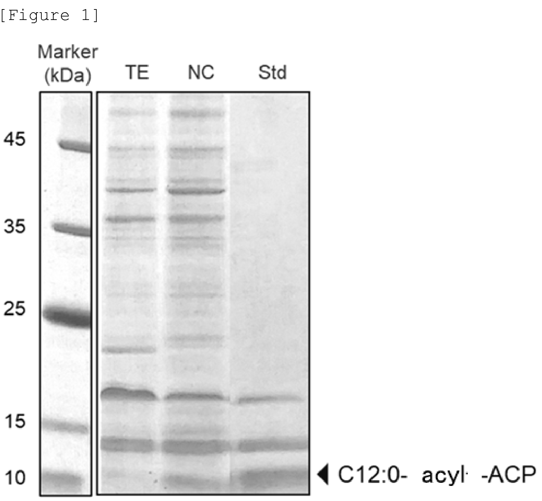

The result of SDS-PAGE obtained for the transformant with the mutant thioesterase gene of *Tepidiphilus thermophilus* is shown in Figure 1. Here, the symbol NC represents the negative control strain transformed with the empty vector, and the symbol Std represents the crude purification liquid of C12:0-acyl-ACP used for the reaction. An about 10-kDa band of C12:0-acyl-ACP was present in strain NC, but disappeared in strain TE. It can be seen that when the DNA of SEQ ID NO: 1 was introduced into strain TH-1, an enzyme functioning as a thioesterase was produced and C12:0-acyl-ACP was decomposed.

### (2-3) Measurement of aliphatic acyl-CoA reductase activity

A disrupted cell solution was prepared for the aliphatic acyl-CoA reductase gene-introduced strain of *Marinobacter lutaoensis* (strain FAR) prepared as described above by the method described in the section "(2-2) Measurement of thioesterase activity." The disrupted cell supernatant was used as a crude enzyme solution, and its aliphatic acyl-CoA reductase activity was measured. A reaction buffer was added to the crude enzyme solution, and 5 mM NADPH serving as a coenzyme and 1 mM lauroyl-CoA or myristoyl-CoA serving as a substrate were mixed thereinto, followed by a reaction at 52°C for 60 minutes. The reaction mixture was analyzed with a gas chromatograph (Shimadzu Corporation: GC-2014, Polar-WAX column), and produced 1-dodecanol or 1-tetradecanol was identified by detecting a peak showing the same column retention time as that of a standard.

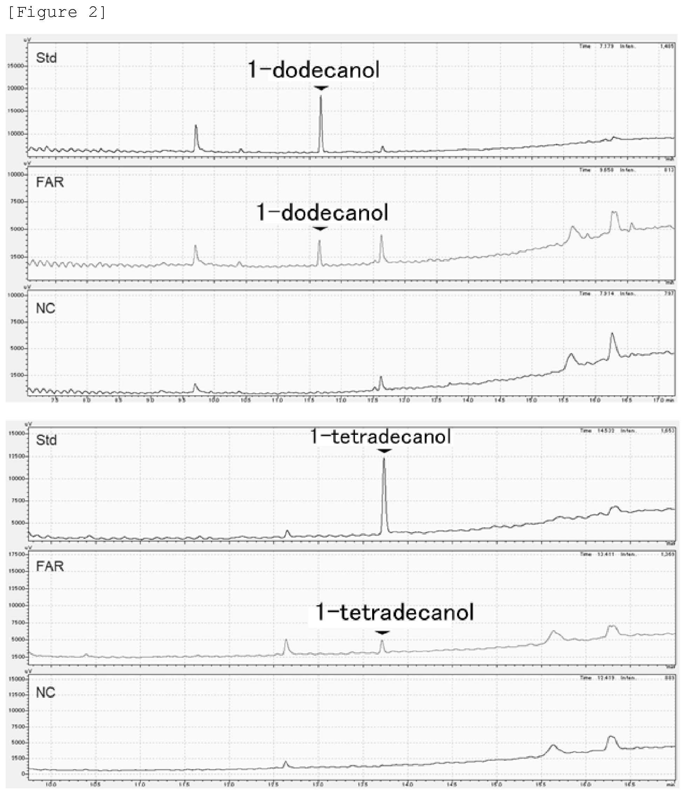

A chromatogram obtained for the transformant with the aliphatic acyl-CoA reductase gene of *Marinobacter lutaoensis* is shown in Figure 2. Herein, the symbol NC represents the negative control strain transformed with the empty vector, and the symbol Std represents a standard of 1-dodecanol and 1-tetradecanol (100 µM). It can be seen that in strain FAR in which the DNA of SEQ ID NO: 3 was introduced into strain TH-1, 1-dodecanol and 1-tetradecanol were produced unlike strain NC, and an enzyme functioning as an aliphatic acyl-CoA reductase was produced.

### (2-4) Production of aliphatic alcohol

Strain FA-1 obtained by introducing both the mutant thioesterase gene of *Tepidiphilus thermophilus* and the aliphatic acyl-CoA reductase gene of *Marinobacter lutaoensis* into wild strain TH-1, and strain NC obtained by introducing the empty vector are each inoculated with a platinum loop into liquid A medium containing 50 µg/mL kanamycin [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O are dissolved in 1 L of distilled water (pH 7.0)], and are each cultured with shaking at 52°C for 24 hours while a gas mixture of H₂:O₂:CO₂ = 7.5:1:1.5 is supplied along with the culture. After the culture, the resultant is centrifuged (4°C, 5,000 g, 10 minutes) to provide a culture supernatant. In the culture supernatant of strain FA-1, 1-dodecanol and 1-tetradecanol are produced unlike the culture supernatant of strain NC into which the empty vector is introduced.

### (3) Production of acyl-CoA dehydrogenase gene-disrupted strain

### (3-1) Acquisition of streptomycin-resistant strain

*Hydrogenophilus thermoluteolus* strain TH-1 was inoculated with a platinum loop into 5 mL of liquid A medium in a test tube, and was cultured with shaking at 52°C after the test tube had been filled with a gas mixture of H₂:O₂:CO₂ = 7.5:1:1.5. The culture liquid after 24 hours was applied to solid LB medium containing 100 µg/ml streptomycin, followed by culture at 52°C for 72 hours.

As a result, the formation of three colonies was able to be recognized on the solid LB medium containing 100 µg/ml streptomycin. Those colonies were inoculated with a platinum loop into 5 mL of liquid A medium containing 100 µg/ml streptomycin in a test tube, and the test tube was filled with a gas mixture of H₂:O₂:CO₂ = 7.5:1:1.5, followed by culture at 52°C. 24 hours after that, the growth of bacteria was observed. Accordingly, those strains were the streptomycin-resistant strains of strain TH-1, and one of the strains was named strain SR88.

### (3-2) Construction of plasmid for gene disruption (pCAMO-7)

Genomic DNA was extracted from the wild strain (streptomycin-sensitive strain) of the strain TH-1 according to a conventional method. Through use of the extracted genomic DNA as a template, a DNA fragment containing an rpsL gene, which was a streptomycin sensitivity gene, encoding ribosomal protein S12, was amplified by a PCR method. The following primers were used for the PCR.

Primers for amplifying wild-type rpsL gene of strain TH-1

| | | |
|---|---|---|
| (a-4) | 5'-CTGGAGGAGAAACGCATATGCCAACCATCAACCAGTTGGTG-3' | (SEQ ID NO: 14) |
| (b-4) | 5'-CGACGGAGCTCGAATTCTTATTTCTTGCCCGCAGCGGC-3' | (SEQ ID NO: 15) |

A sequence homologous to that of vector pCAMO-6 is added to each of the primers (a-4) and (b-4).

The resulting reaction mixture was subjected to gel electrophoresis. As a result, an about 0.4-kbp DNA fragment was detected for the rpsL gene derived from strain TH-1, and was recovered from the gel.

Plasmid vector pCAMO-6 was amplified by PCR through use of the DNA fragment of SEQ ID NO: 7 as a template for subsequent DNA ligation by Gibson Assembly. The primers (a-3) and (b-3) were used for the PCR.

An about 5.2-kbp DNA fragment corresponding to the vector gene was detected, and was recovered from the agarose gel.

The DNA fragment of vector pCAMO-6 synthesized above and the DNA fragment of the rpsL gene were ligated to each other by Gibson Assembly.

*Escherichia coli* strain JM109 was transformed with the resulting reaction mixture by heat shock method, applied to LB medium containing 50 µg/mL kanamycin, and cultured at 37°C for 24 hours.

Each grown strain on the LB medium was inoculated with a platinum loop into 5 mL of liquid LB medium containing 50 µg/mL kanamycin in a test tube, and cultured with shaking at 37°C. Plasmid DNA was extracted from the culture liquid. Analysis of the sequence of the rpsL gene inserted in the plasmid was performed by Sanger method. The results revealed that the sequence was identical to the corresponding sequence in a database. The resulting plasmid was named pCAMO-7. pCAMO-7 has the streptomycin sensitivity gene (rpsL gene) and a kanamycin resistance gene derived from pCAMO-6.

### (3-3) Construction of DNA for disrupting HPTL_0940 gene (pCAMO-8)

A DNA fragment having an HPTL_0940 gene, the 5' upstream region of the HPTL_0940 gene, and the 3' downstream region of the HPTL_0940 gene was amplified by PCR method through use of the genomic DNA of the wild strain of strain TH-1 as a template. The following primers were used for the PCR.
Primers for amplifying DNA fragment containing DNAs of HPTL_0940 gene and surrounding region

| | | |
|---|---|---|
| (a-5) | 5'-GAATCGCGAAATAAGGCCCTTGGTCACGCCACTTGGCACGC-3' | (SEQ ID NO: 16) |
| (b-5) | 5'-CTATCAACAGGAGTCCCACCTTTCATCAACCGGCAAGGGGAG-3' | (SEQ ID NO: 17) |

A sequence homologous to that of vector pCAMO-7 is added to each of the primers (a-5) and (b-5).

The resulting reaction mixture was subjected to gel electrophoresis. As a result, an about 5.9-kbp DNA fragment corresponding to the DNAs of the HPTL_0940 gene, and the upstream region and downstream region of the HPTL_0940 gene was detected, and was recovered from the gel.

Plasmid vector pCAMO-7 was amplified by PCR for subsequent DNA ligation by Gibson Assembly. The following primers were used for the PCR.
Primers for amplifying plasmid vector pCAMO-7

| | | |
|---|---|---|
| (a-6) | 5'-GGACTCCTGTTGATAGATCCAGTAATGACC-3' | (SEQ ID NO: 18) |
| (b-6) | 5'-GGCCTTATTTCGCGATTCCCAAGAAGACAG-3' | (SEQ ID NO: 19) |

The resulting reaction mixture was subjected to gel electrophoresis. As a result, an about 3.4-kbp DNA fragment was detected, and was recovered from the gel.

The DNA fragment of vector pCAMO-7 synthesized above, and a DNA fragment of a gene for disruption containing the HPTL_0940 gene and regions on both sides thereof were ligated to each other by Gibson Assembly.

*Escherichia coli* strain JM109 was transformed with the resulting reaction mixture by heat shock method, applied to LB medium containing 50 µg/mL kanamycin, and cultured at 37°C for 24 hours.

Each grown strain on the LB medium was inoculated with a platinum loop into 5 mL of liquid LB medium containing 50 µg/mL kanamycin in a test tube, and cultured with shaking at 37°C. Plasmid DNA was extracted from the culture liquid. The analysis of the nucleotide sequence of the gene inserted in the plasmid was performed by Sanger method. The results revealed that the sequence was identical to the corresponding sequence in a database.

A DNA fragment of a gene in which HPTL_0940 was deleted was amplified by PCR method through use of the plasmid thus prepared as a template. The following primers were used for the PCR.

| | | |
|---|---|---|
| (a-7) | 5'-GCGCACACCGGGAGCATAGCGTGGCGGATCG-3' | (SEQ ID NO: 20) |
| (b-7) | 5'-CCATCTCCTATCCGAATGGATCGAACGTTTGTTTGAATGATACG-3' | (SEQ ID NO: 21) |

The resulting reaction mixture was subjected to gel electrophoresis. As a result, an about 6.3-kbp DNA fragment was detected. The DNA fragment was ligated by a ligation method using T4 DNA ligase and T4 DNA kinase (manufactured by Takara Bio Inc.) to produce a circular plasmid in which the HPTL_0940 gene was deleted. *Escherichia coli* strain JM109 was transformed with the resulting reaction mixture by heat shock method, was applied to LB medium containing 50 µg/mL kanamycin, and was cultured at 37°C for 24 hours.

Each grown strain on the LB medium was inoculated with a platinum loop into 5 mL of a liquid LB medium containing 50 µg/mL kanamycin in a test tube, and was cultured with shaking at 37°C, followed by the extraction of plasmid DNA from the culture liquid. The nucleotide sequence of the gene inserted into the plasmid was analyzed by Sanger method, and was recognized to be identical to the corresponding sequence on a database.

The resulting plasmid was named pCAMO-8. pCAMO-8 has regions on both sides of the HPTL_0940 gene, and a marker cassette containing a streptomycin sensitivity gene (counterselection marker) and a kanamycin resistance gene (positive selection marker).

### (3-4) Disruption of HPTL_0940 gene (positive selection and counterselection)

A DNA fragment from which the origin of replication of *Escherichia coli* was deleted was amplified by PCR method through use of the plasmid pCAMO-8 for disrupting the HPTL_0940 gene obtained in the section (3-3) as a template and the following primers.

| | | |
|---|---|---|
| (a-8) | 5'-GGCGGAGCCTATGGAAAAACGCC-3' | (SEQ ID NO: 22) |
| (b-8) | 5'-CGGGGTCTGACGCTCAGTGGAAC-3' | (SEQ ID NO: 23) |

The resulting reaction mixture was subjected to gel electrophoresis. As a result, an about 5.7-kbp DNA fragment was detected. The DNA fragment was ligated by a ligation method to prepare a circular plasmid from which the origin of replication of *Escherichia coli* was deleted.

Streptomycin-resistant strain SR88 of *Hydrogenophilus thermoluteolus* was transformed with the resulting reaction mixture by electric pulse method (electroporation method), was applied to solid LB medium containing 50 µg/mL kanamycin, and was cultured at 52°C for 48 hours. The grown gene-introduced strain of *Hydrogenophilus thermoluteolus* was inoculated with a platinum loop into liquid A medium containing 50 µg/mL kanamycin, and was cultured with shaking at 52°C for 24 hours while a gas mixture of H₂:O₂:CO₂ = 7.5:1:1.5 was supplied along with the culture. The introduced circular plasmid lacks the origin of replication. Accordingly, a strain that grows in the kanamycin-containing medium is not a strain that has been grown by the kanamycin resistance gene of the circular plasmid, but a strain that has been grown through the incorporation of the kanamycin resistance gene into the genome of *Hydrogenophilus thermoluteolus* by first homologous recombination. In the first homologous recombination, the entirety of the introduced circular plasmid is incorporated into the genome of *Hydrogenophilus thermoluteolus* by a single crossover.

The culture liquid obtained as a result of the foregoing was applied to solid LB medium containing 100 µg/mL streptomycin, and was cultured at 52°C for 24 hours. A strain grown on the streptomycin-containing medium is a strain in which the 5' upstream region and 3' downstream region of an HPTL_0940 gene region are adjacent to each other as a result of the excision of a marker cassette consisting of the kanamycin resistance gene and the streptomycin sensitivity gene by second homologous recombination between 5' upstream regions or between 3' downstream regions in the genome of *Hydrogenophilus thermoluteolus*, that is, a strain in which the HPTL_0940 gene originally present therebetween has been excised, or a wild type.

A strain in which the HPTL_0940 gene was disrupted was sorted out of the grown strains on the solid LB medium by colony PCR. The colony PCR was performed by a conventional method with the primers (a-5) and (b-5). An about 5.9-kbp DNA fragment is amplified in a wild strain, and an about 3.3-kbp DNA fragment is amplified when the HPTL_0940 gene is deleted. A strain in which the about 3.3-kbp DNA fragment in which the HPTL_0940 gene was deleted was amplified was named strain Δ0940.

### (4) Introduction of mutant thioesterase gene and aliphatic acyl-CoA reductase gene into acyl-CoA dehydrogenase gene-disrupted strain (strain Δ0940)

### (4-1) Gene introduction

The resulting strain Δ0940 is transformed with a plasmid obtained by inserting the mutant thioesterase gene of *Tepidiphilus thermophilus* and the aliphatic acyl-CoA reductase gene of *Marinobacter lutaoensis* into pCAMO-6, is applied to solid LB medium containing 50 µg/mL kanamycin, and is cultured at 52°C for 48 hours. Each grown strain on the solid LB medium is inoculated with a platinum loop into solid LB medium containing 50 µg/mL kanamycin, and is cultured at 52°C for 24 hours. For each of the grown strains on the solid LB medium, the amplification of a DNA fragment having a length corresponding to each gene is recognized by recognizing the amplification of an inserted fragment of the plasmid by PCR. The resulting transformant strain is named strain FA-2.

### (4-2) Production of aliphatic alcohol by transformant strain

The prepared strain FA-2 is inoculated with a platinum loop into liquid A medium containing 50 µg/mL kanamycin, and is cultured with shaking at 52°C for 24 hours while a gas mixture of H₂:O₂:CO₂ = 7.5:1:1.5 is supplied along with the culture. After the culture, a culture supernatant is obtained by centrifugation (4°C, 5,000 g, 10 minutes). In the culture supernatant of strain FA-2, 1-dodecanol and 1-tetradecanol are produced in amounts larger than those in the culture supernatant of strain FA-1 obtained by introducing the mutant thioesterase gene of *Tepidiphilus thermophilus* and the aliphatic acyl-CoA reductase gene of *Marinobacter lutaoensis* into the wild strain of *Hydrogenophilus thermoluteolus* strain TH-1.

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1 to 7 are shown below.
[Chem. 1]
   SEQ ID NO: 1 (Mutant thioesterase gene of *Tepidiphilus thermophilus)* 
ATGGCCACGATTCTCGTCTTCGGCGACAGTCTCTCCGCGGCTATGGCTACCGCCGCAAAGCGGCTGGCCCTA
CCTGCTGCAGCGCCAGCTCGATGCGCTCGAACCTGGAGCACACCGCGTCATCGACGCCAGCGTCTCGGGGAGA
CCACGGCCGGGGGAGCCTCCCGCCTGCCGAGGCTGCTCGAACGGCACCGACCCGATGTCGTCGTGCTCGCCCTG
GGCGCCAACGATGGCCTGCGCGGCCTGCCGCCGAAAGCGATGGAGACCAATCTCGAGCGCATGATCCTCATGGC
CAAACAGTCCGGCTGCGAAGTCGTGCTCGTGGGCATGAGACTGCCCCCCAATCTGGGGCCCGATTACACTGCCG
AGTTCGATGCGGTCTATCCGCGCCTCGCAGCCCGTTACCCCGTGCATTTCGTCCCCTTCCTGCTCGAAGCGATC
GCGCTCGACCCTGATGCGTTCCAGGAAGACGGGCTGCACCCGGTGCTGGAGGCCGTGCCCAAGCTGTCCCAGAC
GGTGTGGCCGGCGATCGCCTCGGCGCTGGAAGCGGTGCGCAAACGCGGCGCCGCCTCCGGGCGCTGA
[Chem. 2]
   SEQ ID NO: 2 (Mutant thioesterase of *Tepidiphilus thermophilus)* 
MATILVFGDSLSAGYGLPPQSGWPYLLQRQLDALEPGAHRVIDASVSGETTAGGASRLPRLLERHRPDVVVLAL
GANDGLRGLPPKAMETKLERMILMAKQSGCEVVLVGMRLPPNLGPDYTAEFDAVYPRLAARYPVHFVPFLLEAI
ALDPDAFQEDGLHPVLEAVPKLSQTVWPAIASALEAVRKRGAASGR
[Chem. 3]
   SEQ ID NO: 3 (Aliphatic acyl-CoA reductase gene of *Marinobacter lutaoensis)* 
ATGGCAACACCGCATGTATCCACCGAGTCGTCCTCGAACGTCCTGGAAACGCTTCGGGGCAAGCACGTTCTGAT
CACCGGCACCACTGGGTTCCTGGGCAAGGTGGTTCTGGAAAAACTGATCCGTGCGGTGCCGGACATCGGCGGCA
TTCACCTGCTGATCCGTGGGAACAAGCGGCATCCGGATGCCCGCAGCCGGTTTCTGAATGAAGTGGCGACCTCG
TCCGTATTTGAACGCCTGAGGGCGGAGGACAACGACGGGTTCGAGGCGTTTCTGGAGGAGCGGGTCCATTGCGT
TACCGGGGAAGTCACCGAAGCCCGGTTTGGTCTGGCCGATGCCGAGTTCAGGGCCCTGGCCGGCCGCTTGGACG
CCGTCATCAACTCCGCGGCGAGTGTCAACTTCCGCGAGGAACTCGACAAGGCACTGGCCATCAACACTCTGTGC
CTGCACAACATCGCCGATCTGGCTGCGCGCAATCCTGACCTGGCGGTTATTCAGGTCTCCACCTGCTACGTCAA
TGGCATGAACTCGGGGCAAGTGACGGAATCCGTCATCAAGCCGGCGGGGGAAGCCATACCCCGGACCGCACACG
GCTACTACGAAATCTCCGAGTTGGTGCGTCTGCTGCAGGACAAGATCGCCGACGTCCGCTCCCGATACTCCGGC
AAAACTCTGGAAAAGAAACTGGTGGAGCTGGGCATCCGCGAGGCCAACCGCTACGGCTGGAGCGACACCTACAC
CTTCACCAAGTGGTTGGGCGAACAACTGTTGATGAAGGCACTTGCCGGCCGGACTCTGACCATTGTCCGGCCCT
CCATCATTGAGAGCGCTCTGGAAGAACCGGTGCCGGGCTGGATCGAGGGCGTTAAGGTGGCGGACGCCATCATC
CTGGCCTATGCCCGGGAGAAGGTGACCCTGTTCCCCGGCAAGCGCTCCGGCGTGATCGACGTGATCCCGGTGGA
CCTGGTCGCCAACGCCATTGTCCTGGCCTTGGCCGAGGCGTTGTCGGCTCCCGCCGGCCCGCGTGTGTACCAGT
GCTGCAGCGGCAGCTCCAACCCCATTTCCCTGGGGCAGTTCATCGACCACCTGATGGCGGAGTCGAAAGCCAAC
TATGCCGCCTATGACTCGCTGTTCTACCGGCAGCCCACCAAGCCGTTCGTGGCCGTCAACCGACGCCTGTTCGA
TGCCATCATGGGGGGCGTGCGTCTGCCCCTGAGCCTGGCCGACCGGGTCTTCAGGCTGCTGGGGCAGAGCCGGG
AGCTGAAGATGCTGCGCAATCTGGATACCACCCGCTCGCTGGCCACCATCTTCGGTTTTTACACGGCGCCCGAT
TACATCTTCCGCAACGACGAGTTGCTGGCCCTGTCGGCCCGGATGGGGGCCGTGGATCAGGCCCTGTTCCCGGT
GGATGCCCGTCGCATCGACTGGTCCGTCTACCTGCGCAAGATCCACCTGGCGGGGCTGAACCGCTACGCCCTGA
AGGAGCGCAAGCTGTACAGCCTGCGCTCCGCCAAGCGAGAAAGAAACAGGCCGCCTGA
[Chem. 4]
   SEQ ID NO: 4 (Aliphatic acyl-CoA reductase of *Marinobacter lutaoensis)* 
MATPHVSTESSSNVLETLRGKHVLITGTTGFLGKVVLEKLIRAVPDIGGIHLLIRGNKRHPDARSRFLNEVATS
SVFERLRAEDNDGFEAFLEERVHCVTGEVTEARFGLADAEFRALAGRLDAVINSAASVNFREELDKALAINTLC
LHNIADLAARNPDLAVIQVSTCYVNGMNSGQVTESVIKPAGEAIPRTAHGYYEISELVRLLQDKIADVRSRYSG
KTLEKKLVELGIREANRYGWSDTYTFTKWLGEQLLMKALAGRTLTIVRPSIIESALEEPVPGWIEGVKVADAII
LAYAREKVTLFPGKRSGVIDVIPVDLVANAIVLALAEALSAPAGPRVYQCCSGSSNPISLGQFIDHLMAESKAN
YAAYDSLFYRQPTKPFVAVNRRLFDAIMGGVRLPLSLADRVFRLLGQSRELKMLRNLDTTRSLATIFGFYTAPD
YIFRNDELLALSARMGAVDQALFPVDARRIDWSVYLRKIHLAGLNRYALKERKLYSLRSAKARKKQAA
[Chem. 5]
   SEQ ID NO: 5 (HPTL_0940 gene of *Hydrogenophilus thermoluteolus*) 
ATGGCTTGGCTCTTGTTGCTCTTCCTCCCTCCCTTGGTGGTCGCGCTCGCCTACGGGCGAGCGCCTCTTTTTCT
CTGGGGTGGAGCGATCCTGGTTTGGTGGTTGGCGTTGGCCATCACCGCAGCGTTTCCGGTATGGGTCACGGTTG
TCGTGGCGCTTGCCTGGTGTGGCGTGGTCTTTTTGTTGGGGCATCGGCCGACGCGCAAACGTCTGATCACGGTG
CCCATTTTCAAAGCGTTTGCGAAAGCGCTGCCGCAAATGTCGCAGACGGAGCGGGATGCGCTTGAGGCGGGCAC
CGTTTGGTGGGAAGGGGAGCTCTTTCGTGGGAACCCGGATTGGCAAAAATTCCTGAGCTACCCGTGGCCGAAAC
TGACGCCGGAAGAGCAAGCGTTCCTCGAAAACGAAACCGAGGAGCTCTGCCGCATGGTCGACGATTGGGAGTGC
AGCCTCAAGAAGGACATGCCCGATGCCGTCTGGCAGTTCATCAAGGAAAAGGGGTTCCTCGGGATGATCATCCC
GAAAGAGTATGGCGGGAAAGGTTTTTCCGCGTACGCCCATTCGCAGGTGATCACCAAACTCTCGACCCGTTCGA
GCGCTCCGGCAGTGACCGTGATGGTGCCCAATTCGTTGGGGCCTGCAGAGCTGCTGCTCCACTATGGACGCCG
GAACAGAAAGCCTACTACTTGCCGCGCCTGGCGCGCGGGAAGAGATTCCCGCATTTGCGCTCACCAGTCCTTG
GGCCGGTTCGGACGCCGCGTCGATTCCCGATGCCGGTGTCGTCTGCCGCGGCGAATACCAGGGCAAAGAGGTGA
TCGGTATTCGCGTGAGCTTCAACAAGCGCTATATCACGCTCGCGCCGGTGTGTACGGTCTTCGGGCTGGCGTTC
CAGCTTTTTGACCCGGAAAACTTGTTGGGTAGAGGGAAAGAGATCGGGATTACGCTCGCGCTCATTCCGGCAAA
CCATCCGGGCGTCGAAATCGGGCGTCGGCACCTTCCACTCGACGCGGTGTGGATGAACGGACCGATCCGCGGGG
AAGAGGTCTTCATTCCGCTCGATTTCGTGATCGGTGGTGAGGCGATGGTGGGCAAAGGGTGGCGGATGCTGATG
GAGTGCCTGGCGGCTGGGCGCTCGATCTCGTTGCCCGGCTCCAACACTGGGATGCAGCAGGTCACGGCGCGCGC
GGTCGGTGCGTACGCGCGGATCCGATACCAATTCAAGACGCAGATCGGTCGCTTCGAAGGGGTGGAAGAGGCGC
TCGCGCGCATCGGTGGCAACACCTACCTGTGTGACGCTGCGCGGGTATTTACTGCTGGCGCGGTCGATCTCGGT
GAGAAGCCGTCGGTGGTTTCGGCGATCGTCAAATACCACGTGACCGAACGGCACGCCAGACCGTCAACGACGG
GATGGACGTGGTCGGTGGTAAAGGAATCTGTTTGGGTCCGCAAAACTTCCTGGGGCGGGCGTACGAGCAGATTC
CCGTGGGGATCACCGTCGAAGGGGCGAACATCCTCACCCGCAGCCTCATCGTTTTCGGGCAAGGGGCGGTTCGG
TGCCATCCCTACGTGCTCGAAGAGATGCGTGCTGCGCAAAACGGTGACCTCGATGCGTTCGACCGCGCGTTCTG
GAGTCACATCGCCTATACGCTCAACAACGGTGCCCGTGCGCTGGTGATGGGGCTTACTGGGGCCCGTTTCGTGA
AGGTGCCAGCAAACGTTGCGCCGGAGTGCCGTCGCTATTACCAACAGCTGACCCGTTTTTCCGCAGCGTTCGCG
TTCTTGGCCGATGTCTCGATGGGCGTGTTGGGGGGTGCGCTGAAACGTAAAGAGCGGCTCTCTGCCCGTTTGGG
TGACATTTTGTCGCAGCTCTACTTGGCGAGCGCTACGCTCAAGCGCTTCGAAGTCGACGGCCGCCCCGCTGGTG
ATGCGCCGTTCCTGCACTGGGCGATCTGGGATGCGTTTTTCCGCGCTCAAGTGGCCTTTACTGGCGTGATCGAC
AATTTCCCATTCGCGCCGGCGCGGGCGCTGGCGCGGTTCGTCACCTTCCCGCTGGGTCGTCCGTTCCGTACGCC
GGACGATCGTCTTGGACATCGGGTGGCGATGGGTCTGATGGAGCCCGGTGCAACCCGTGACCGTTTGACCTCGC
TCGTCTATTTGCCCCAATCGCTCGACGAGCCGCTGGGGGCGTTGGAAGCAGCACTCTTGGCCACCCAAGCGTGT
GAGCCGATCGAAGCGAAATTGCGCGACGCGCAGAAAGCGGGACGCTTTGCACCGAGCATCGAAGCACTCACCAG
CGGCAGTGTCGATGCGATTTGGGATGAAGCGTATGCGCAAGGGGTGATCACCGAGGCCGAATACCGCAAAGTGA
TCCAGCGCAATCGGCTGCGTGACCTGGTGATCCGTGTCGATGACTTCCCCTACGATTTTGGGGTCAAGGCGGCC
GCGCAGGCGATTCCGGACGATCGTATCGCGCGCACCGCGGCCATTCCGCCCGCGATGCGTCCGTGGCCGGAAAA
GCGGCGCGTCGGAGGGGATGTGGCGGTGCAGGATGCGCCAGCGGTTTCGGCTTCGACCGTCGATGCGTAA
[Chem. 6]
   SEQ ID NO: 6
   (Polypeptide encoded by HPTL_0940 gene of *Hydrogenophilus thermoluteolus)* 
MAWLLLLFLPPLVVALAYGRAPLFLWGGAILVWWLALAITAAFPVWVTVVVALAWCGVVFLLGHRPTRKRLITV
PIFKAFAKALPQMSQTERDALEAGTVWWEGELFRGXPDWQKFLSYPWPKLTPEEQAFLENETEELCRMVDDWEC
SLKKDMPDAVWQFIKEKGFLGMIIPKEYGGKGFSAYAHSQVITKLSTRSSAPAVTVMVPNSLGPAELLLHYGTP
EQKAYYLPRLARGEEIPAFALTSPWAGSDAASIPDAGVVCRGEYQGKEVIGIRVSFNKRYITLAPVCTVFGLAF
QLFDPENLLGRGKEIGITLALIPANHPGVEIGRRHLPLDAVWMNGPIRGEEVFIPLDFVIGGEAMVGKGWRMLM
ECLAAGRSISLPGSNTGMQQVTARAVGAYARIRYQFKTQIGRFEGVEEALARIGGXTYLCDAARVFTAGAVDLG
EKPSVVSAIVKYHVTERARQTVNDGMDVVGGKGICLGPQNFLGRAYEQIPVGITVEGANILTRSLIVFGQGAVR
CHPYVLEEMRAAQNGDLDAFDRAFWSHIAYTLXNGARALVMGLTGARFVKVPANVAPECRRYYQQLTRFSAAFA
FLADVSMGVLGGALKRKERLSARLGDILSQLYLASATLKRFEVDGRPAGDAPFLHWAIWDAFFRAQVAFTGVID
NFPFAPARALARFVTFPLGRPFRTPDDRLGHRVAMGLMEPGATRDRLTSLVYLPQSLDEPLGALEAALLATQAC
EPIEAKLRDAQKAGRFAPSIEALTSGSVDAIWDEAYAQGVITEAEYRKVIQRNRLRDLVIRVDDFPYDFGVKAA
AQAIPDDRIARTAAIPPAMRPWPEKRRVGGDVAVQDAPAVSASTVDA
[Chem. 7]
   SEQ ID NO: 7 (pCAMO-6) Former half 
CCCATCGACTGCACGGTGCACCAATGCTTCTGGCGTCAGGCAGCCATCGGAAGCTGTGGTATGGCTGTGCAGGT
CGTAAATCACTGCATAATTCGTGTCGCTCAAGGCGCACTCCCGTTCTGGATAATGTTTTTTGCGCCGACATCAT
AACGGTTCTGGCAAATATTCTGAAATGAGCTGTTGACAATTAATCATCGGCTCGTATAATGTGTGGAATTGTGA
GCGGATAACAATTTCACACAGGAAACAGGCCAGTTAGGCCTGCTAGCAGATCTGGAGGAGAAACGCATATGCTG
ATATCGGATCCGAATTCGAGCTCCGTCGACAAGCTTGCGGCCGCACTGCAGCACCATCACCACCATCATTGATA
AGATCCGGCTGCTAACAAAGCCCGAAAGGAAGCTGAGTTGGCTGCTGCCACCGCTGAGCAATAACTCGAGGCCA
ATGAGGCCTTCAAATAAAACGAAAGGCTCAGTCGAAAGACTGGGCCTTTCGTTTTATCTGTTGTTTGTCGGTGA
ACGCTCTCCTGAGTAGGACAAATCCGCCGGGAGCGGATTTGAACGTTGCGAAGCAACGGCCCGGAGGGTGGCGG
GCAGGACGCCCGCCATAAACTGCCAGGCATCAAATTAAGCAGAAGGCCATCCTGACGGATGGCCTTTTTGCGTT
TCTACAAACTCCGCGGATCTAGGTGAAGATCCTTTTTGATAATCTCATGACCAAAATCCCTTAACGTGAGTTTT
CGTTCCACTGAGCGTCAGACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATCAAGAGCTACCAACTCTTTT
TCCGAAGGTAACTGGCTTCAGCAGAGCGCAGATACCAAATACTGTCCTTCTAGTGTAGCCGTAGTTAGGCCACC
ACTTCAAGAACTCTGTAGCACCGCCTACATACCTCGCTCTGCTAATCCTGTTACCAGTGGCTGCTGCCAGTGGC
GATAAGTCGTGTCTTACCGGGTTGGACTCAAGACGATAGTTACCGGATAAGGCGCAGCGGTCGGGCTGAACGGG
GGGTTCGTGCACACAGCCCAGCTTGGAGCGAACGACCTACACCGAACTGAGATACCTACAGCGTGAGCTATGAG
AAAGCGCCACGCTTCCCGAAGGGAGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGCAACGCGGCCTTTTTACGGTTCC
TGGCCTTTTGCTGGCCGCGGACGTAGAAAGCCAGTCCGCAGAAACGGTGCTGACCCCGGATGAATGTCAGCTAC
TGGGCTATCTGGACAAGGGAAAACGCAAGCGCAAAGAGAAAGCAGGTAGCTTGCAGTGGGCTTACATGGCGATA
GCTAGACTGGGCGGTTTTATGGACAGCAAGCGAACCGGAATTGCCAGCTGGGGCGCCCTCTGGTAAGGTTGGGA
AGCCCTGCAAAGTAAACTGGATGGCTTTCTTGCCGCCAAGGATCTGATGGCGCAGGGGATCAAGATCCGATCAA
GAGAGGCCACTAAGGCCCAGGATGAGGATCGTTTCGCATGATTGAACAAGATGGATTGCACGCAGGTTCTCCGG
CCGCTTGGGTGGAGAGGCTATTCGGCTATGACTGGGCACAACAGACAATCGGCTGCTCTGATGCCGCCGTGTTC
CGGCTGTCAGCGCAGGGGCGCCCGGTTCTTTTTGTCAAGACCGACCTGTCCGGTGCCCTGAATGAACTACAGGA
CGAGGCAGCGCGGCTATCGTGGCTGGCCACGACGGGCGTTCCTTGCGCAGCTGTGCTCGACGTTGTCACTGAAG
CGGGAAGGGACTGGCTGCTATTGGGCGAAGTGCCGGGGCAGGATCTCCTGTCATCTCACCTTGCTCCTGCCGAG
AAAGTATCCATCATGGCTGATGCAATGCGGCGGCTGCATACGCTTGATCCGGCTACCTGCCCATTCGACCACCA
AGCGAAACATCGCATCGAGCGAGCACGTACTCGGATGGAAGCCGGTCTTGTCGATCAGGATGATCTGGACGAAG
AGCATCAGGGGCTCGCGCCAGCCGAACTGTTCGCCAGGCTCAAGGCGCGTATGCCCGACGGCGAGGATCTCGTC
GTGACTCATGGCGATGCCTGCTTGCCGAATATCATGGTGGAAAATGGCCGCTTTTCTGGATTCATCGACTGTGG
CCGGCTGGGTGTGGCGGACCGCTATCAGGACATAGCGTTGGCTACCCGTGATATTGCTGAAGAGCTTGGCGGCG
AATGGGCTGACCGCTTCCTCGTGCTTTACGGTATCGCCGCTCCCGATTCGCAGCGCATCGCCTTCTATCGCCTT
CTTGACGAGTTCTTCTGAGGCCATCTAGGCCATGAGTAAGTCTGACGGAACGGAAAGTTGTCGGTTACCAGCAA
CGTTTCGCCCTGTTGGCCTCAAAAGTTCGAGTCCGTTATTGGTATTTCCATACCAACTCAAACGGGATATAATG
TGACATGTAACATTTCTCGATTGAGGAACGACAATGACAGTACCGGTCAAAGCGCGTGTTCAAAAGTATCGCGA
CGCGCTGCGCCGCTCGGGTTTGCGTCCGGTTCAGATATGGGTGCCGGATACCCGCCGCCCGGGCTTTGCACAAG
[Chem. 8]
   SEQ ID NO: 7 (pCAMO-6) Latter half

AGTGTCGCCGCCAGTGCCTGCTCGTGACCCAGGCAGACGCCGCTGATACGCAACTCCAACGACTCATGGATGAA
GCCTTGGCAGAGATTGATGGCTGGCAGGCATGATGCGCGGCGATTTGGTAACGATCGTTACCCAGGGCGACTTT
GGCAAACCAAGGCCAGCGTTGGTGATCCAGTCCGATCTGTTCAACGAGCTCAGCAGCATAACCGTCTTTCCTGT
AACCAGTACCATTGTGGATGCCCCGTTGTTGCGCGTCACGGTTGAGCCAACCCCGGAAAACGGCTTGCACAAGC
GATCACAGGTGATGGTGGACAAAATTATGACCGTGCGGCGGACAAAGTCGGTCAGATCATTGGACGTATCGAT
ACGACTACGCTGACAACGATCGAGCGCTGCTTAGCTGTCTTCTTGGGAATCGCGAAATAAGGCCATGAAGGCCT
TACCCATCACCGTGCATCACGCCGACCGCTTCGCCACTTTGCGACAAGAAAGCAATCTGAACGCGCTGGCGAAA
ATGGATCGGCGCTGCCCGGTCTACCTGTGAAGTTCGCGCAAACGTCCATCGAGACGTACATTGCCGTTGGCATC
GATCTCGACGGCGATTTCCTCTGGCTACCGATCGCGCGCACCGCTTACGCGCCCTCGACGTAGTCGAGAAAAGC
GTGATTGCGTATGTTGCGCCAACTGAGCCCGTCGGTGCGTTGTGACCACTCGTTGTAGACCACGCCCAAAACCA
TCACCCCAAACGGTGCGGAAAGTGCTACTCTTTCGTCTTGCCACAACAAGAAGGAGGGCGGGTGGTGATCGAAA
CCGGTGCTTTGAGAGCCAAAGAAGACCCCATTGCCTGGGTCCAACAGAAAGCAGCCGAGGCGTTGCAACGCCTA
GATCAAAAGATGGCCTCTCAGCCCGTGCAGATGTACCTACCGGGGTTCGATCTAGGGGCCTTTCCCAACCATGT
GAACCGCTCCTCGCTCTTTGCGCCTATTGCGCGCGGTCGCCGCAAATTTCATCGACAAACGAAGATGGTCAGCC
GCTCGGATGCTGTGATCGAATACACTGGAGAGCAGCTCGACGAAGCCGACTGCGACCTCATGATGGCGCTCATC
TGGTTTGCCCAGAAAAAACCGTTAGGGGAGTGGGTGACATTGAACCGAGCGCAGCTGTTGCGGCGGATCGGGCG
CGCGACGGGCAAGCAGAACTACGAGTGGCTCCATCGCAGAATGCGGGCGATGACCGAAGCGACACTCTACATCG
AAGTGCGCAAATCCGACGGCAGCACACGCTACAAGATCGGCTACGCCGAGGCGTTTCACCTCATCAGCGGCTTT
GCGTTCGATGAGAAAAGCGACGAATACTGCTACCAACTCGATCCCCGCTGGTACAGCTCTTCAGCAACCGCGA
ATACAGCCTGATCAACTTCGACTTGCGATTGCAGATCAAACGGGGTCAGGACATGGCGAAAACCCTGCAACGCC
TCGTTGCTACTTCTTCCGACCCCATCCAGTCTTACGAACTCGAATGGCTCAAGAGCAAAATGGAATACACCGGA
AGAATGCGCGATTTCAAAGAGTCGCTCTCCAAGGCGTGCAATGAGCTGAAACGGTTGCACATCGTCAGTGAATA
CAAGATCACTCTGGACAAAAAGAAGCAACGGGAGCTGTTGAAGCTCTGGATTCCCAAAGCGATCACGGCATAAA
AGCGGTGCGACACTGCACGATCAGAAAGTATTTTCAGTTACTCAAACACCGTTTTTGTTGAAAAACAACGAAT
TGTCATCGGTATAGCGTCGCGCACCCGAGACGACCGACGGTGTCTGGCCATCGGTATAGCGTCGCGCTTTCTCC
ACCTCTTGAACAGTCGTTCGGCATCGGTATAGCGTCGCGCCTGCATCGGTATAGCGTCGCGCTTTTTCGGTATA
GCGTCGCGCTTTTTCGGTATAGCGTCGCGCGGTTGTCAAAAAATCTATTATTATTCAATAAGTTACAAGCGGTT
TTTGACCGTCTACTATATTTCTACTTATTTCTACTAGGAAGCGCCTGTGGATAACTTCGCTCTTTTCTACACAG
CCCCTCAGCGGCGCAAGCGCGCCGCTGGGTAGAAAATTCCTATCGCAACGAACTGTCGTTCCTAAAAATAGGCT
ACTCGGTAAAACAACCGTATAAATGATGCCGCGTGCTCGGAGTCAACTCCAGAACGGCATCGGTATAGTGTCGC
ACTTAGCAATGTGCGAATATTTCATCGTTGAGCCGACTTTTGGAGATTGGTCATGGGAGATCCTCGGGTTGTCG
AAGCCTTGGACTCCTGTTGATAGATCCAGTAATGACCTCAGAACTCCATCTGGATTTGTTCAGAACGCTCGGTT
GCCGCCGGGCGTTTTTTATTGGTGAGAATCCAGTCTAGA

### Industrial Applicability

An aliphatic alcohol, which is a raw material highly demanded in cosmetics, detergents, and other oleochemical product industries, can be produced with high efficiency with the transformant of the present invention through use of carbon dioxide as the sole carbon source. Accordingly, the transformant contributes to industrially producing chemical products with high efficiency while solving global warming due to increased atmospheric carbon dioxide.

## Claims

1. A transformant obtained by introducing, into a *Hydrogenophilus* bacterium, a gene encoding a thioesterase that produces a fatty acid from an acyl-ACP and a gene encoding an aliphatic acyl-CoA reductase that produces an aliphatic alcohol from an aliphatic acyl-CoA.

2. The transformant according to claim 1, wherein the gene encoding the thioesterase that produces a fatty acid from an acyl-ACP is the following DNA (a1), (a2), (a3), (a4), or (a5).
(a1) A DNA comprising the nucleotide sequence of SEQ ID NO: 1
(a2) A DNA comprising a nucleotide sequence that has 90% or more identity to SEQ ID NO: 1 and encoding a polypeptide having thioesterase activity for producing a fatty acid from an acyl-ACP (a3) A DNA encoding a polypeptide comprising the amino acid sequence of SEQ ID NO: 2
(a4) A DNA encoding a polypeptide comprising an amino acid sequence that has 90% or more identity to SEQ ID NO: 2, the polypeptide having thioesterase activity for producing a fatty acid from an acyl-ACP
(a5) A DNA encoding a polypeptide comprising an amino acid sequence that has 1 to 20 amino acids deleted, substituted, inserted, or added in the amino acid sequence of SEQ ID NO: 2, the polypeptide having thioesterase activity for producing a fatty acid from an acyl-ACP

3. The transformant according to claim 1 or 2, wherein the gene encoding the aliphatic acyl-CoA reductase that produces an aliphatic alcohol from an aliphatic acyl-CoA is the following DNA (b1), (b2), (b3), (b4), or (b5).
(b1) A DNA comprising the nucleotide sequence of SEQ ID NO: 3
(b2) A DNA comprising a nucleotide sequence that has 90% or more identity to SEQ ID NO: 3 and encoding a polypeptide having aliphatic acyl-CoA reductase activity for producing an aliphatic alcohol from an aliphatic acyl-CoA
(b3) A DNA encoding a polypeptide comprising the amino acid sequence of SEQ ID NO: 4
(b4) A DNA encoding a polypeptide comprising an amino acid sequence that has 90% or more identity to SEQ ID NO: 4, the polypeptide having aliphatic acyl-CoA reductase activity for producing an aliphatic alcohol from an aliphatic acyl-CoA
(b5) A DNA encoding a polypeptide comprising an amino acid sequence that has 1 to 50 amino acids deleted, substituted, inserted, or added in the amino acid sequence of SEQ ID NO: 4, the polypeptide having aliphatic acyl-CoA reductase activity for producing an aliphatic alcohol from an aliphatic acyl-CoA

4. The transformant according to any one of claims 1 to 3, wherein an acyl-CoA dehydrogenase gene on a chromosome of the *Hydrogenophilus* bacterium is disrupted.

5. The transformant according to claim 4, wherein the acyl-CoA dehydrogenase gene is the following DNA (c1), (c2), (c3), (c4), or (c5).
(c1) A DNA comprising the nucleotide sequence of SEQ ID NO: 5
(c2) A DNA comprising a nucleotide sequence that has 90% or more identity to SEQ ID NO: 5 and encoding a polypeptide having acyl-CoA dehydrogenase activity
(c3) A DNA encoding a polypeptide comprising the amino acid sequence of SEQ ID NO: 6
(c4) A DNA encoding a polypeptide comprising an amino acid sequence that has 90% or more identity to SEQ ID NO: 6, the polypeptide having acyl-CoA dehydrogenase activity
(c5) A DNA encoding a polypeptide comprising an amino acid sequence that has 1 to 80 amino acids deleted, substituted, inserted, or added in the amino acid sequence of SEQ ID NO: 6, the polypeptide having acyl-CoA dehydrogenase activity

6. The transformant according to any one of claims 1 to 5, wherein the *Hydrogenophilus* bacterium is *Hydrogenophilus thermoluteolus*.

7. A method of producing an aliphatic alcohol, comprising a step of culturing the transformant according to any one of claims 1 to 6.
